# EUROPEAN PATENT APPLICATION

(11) **EP 4 778 912 A1**
(43) Date of publication of application: **22.07.2026**
(21) Application number: 23952341.8
(22) Date of filing: 13.09.2023
(51) Int. Cl.: C07C 51/60, C07C 51/64, C07C 63/30

(54) **METHOD FOR PREPARING TEREPHTHALOYL CHLORIDE THROUGH TEMPERATURE CONTROL**

(71) Applicant: Aekyung Chemical Co., Ltd., Seoul 04051 (KR)
(72) Inventor: KIM, Byung Jo, Daejeon 34023 (KR); SHIN, Chul, Seoul 08202 (KR); LEE, Ho Chang, Daejeon 34061 (KR); KIM, Suhan, Daejeon 34087 (KR); KANG, Dong Yun, Daejeon 34116 (KR); OH, Seunghyun, Daejeon 34107 (KR)
(74) Representative: Bassil, Nicholas Charles
(86) International application number: PCT/KR2023/013679
(87) International publication number: WO 2025/058101

(57) **Abstract**

The present invention relates to a method for preparing terephthaloyl chloride and, specifically, to a method for preparing terephthaloyl chloride, the method comprising: preparing hexachloro-p-xylene by performing a reaction in a plurality of temperature sections, specifically, in the fourth or higher temperature sections, according to the conversion rate; and reacting the prepared hexachloro-p-xylene with phthalic acid so as to prepare terephthaloyl chloride.

## Description

### [Technical Field]

The present disclosure relates to a method for producing terephthaloyl chloride through temperature control.

### [Background Art]

Terephthaloyl chloride (TPC) is a white solid or a colorless needle-shaped crystal, and is mainly used as a polymer monomer for poly(paraphenylene terephthalamide) and polysulfonamide.

In addition, terephthaloyl chloride is used as a compatibilizer for polymers and has wide potential for development and application as an intermediate in agrochemical and pharmaceutical industries.

As a method for producing terephthaloyl chloride having various applications as described above, it is known that terephthaloyl chloride (also referred to as terephthaloyl dichloride) is produced by a method in which p-xylene and chlorine gas are subjected to a photo-reaction using ultraviolet light, and hexachloro-p-xylene generated by the photo-reaction is reacted with terephthalic acid in the presence of a catalyst.

However, as the concentration of hexachloro-p-xylene generated in the photo-reaction increases, the viscosity becomes higher, which may cause a decrease in the reaction rate. Accordingly, when the reaction is performed at a high temperature in order to lower the viscosity, the solubility of chlorine gas in liquid p-xylene becomes extremely low, and thus the time for the chlorine gas to participate in the reaction becomes extremely short, which may cause a problem in that the chlorine gas is discharged to the outside before reacting with the p-xylene.

In addition, when p-xylene and chlorine gas are subjected to a photo-reaction, unreacted chlorine gas is discharged together with hydrogen chloride, which is a by-product, thereby causing environmental pollution. That is, hydrogen chloride, which is a by-product, may be easily removed by dissolving it in water as described above, whereas unreacted chlorine gas requires excessive facilities such as those for recovery or storage when discharged to the outside. Therefore, there is a need for a new method in which chlorine gas is completely circulated and consumed in the reaction.

### [Disclosure]

### [Technical Problem]

An object of the present disclosure is to provide a method for producing hexachloro-p-xylene that controls a temperature of a photo-reaction inside a reactor so as to maintain a relatively high solubility of chlorine gas within an appropriate viscosity range in the production of hexachloro-p-xylene.

Another object of the present disclosure is to provide a method for producing hexachloro-p-xylene that prevents chlorine gas from being discharged to the outside by completely consuming unreacted chlorine gas in a reaction without discharging unreacted chlorine gas to the outside.

Still another object of the present disclosure is to provide terephthaloyl chloride by reacting the produced hexachloro-p-xylene with terephthalic acid.

### [Technical Solution]

In one general aspect, there is provided a method for producing hexachloro-p-xylene by subjecting p-xylene and chlorine gas to a photo-reaction, wherein the photo-reaction is performed in a plurality of four or more temperature ranges in accordance with an increase in the melting point of an intermediate product generated depending on the conversion rate of hexachloro-p-xylene.

According to an example embodiment of the present disclosure, in the method for producing hexachloro-p-xylene, a plurality of reactors may be connected in parallel.

According to an example embodiment of the present disclosure, the plurality of reactors may include a first reactor and a second reactor connected in parallel, and a process may be repeatedly performed to produce hexachloro-p-xylene without discharging unreacted chlorine gas to the outside, the process including: continuously introducing chlorine gas into the first reactor charged with p-xylene to initiate a reaction while discharging hydrogen chloride gas that is a by-product generated according to conversion of hexachloro-p-xylene to the outside; transferring, at a conversion rate of hexachloro-p-xylene at which chlorine gas that is continuously introduced into the first reactor in which the reaction is initiated begins to be discharged as an unreacted material, unreacted chlorine gas that is continuously introduced into the first reactor and does not participate in the reaction to the second reactor without being discharged to the outside, so as to allow the transferred unreacted chlorine gas to first react with p-xylene charged into the second reactor; after the reaction in the first reactor is terminated, connecting the chlorine gas that is continuously introduced to the first reactor to the second reactor so as to start continuous introduction of the chlorine gas into the second reactor; and while the first reactor from which the product is discharged is charged with p-xylene, at a conversion rate of hexachloro-p-xylene at which chlorine gas that is continuously introduced into the second reactor begins to be discharged as an unreacted material, transferring the unreacted chlorine gas to the first reactor so as to allow the transferred unreacted chlorine gas to first react with the p-xylene charged into the first reactor.

In another general aspect, a method for producing terephthaloyl chloride by reacting hexachloro-p-xylene with terephthalic acid includes: producing hexachloro-p-xylene by subjecting p-xylene and chlorine gas to a photo-reaction in four or more temperature ranges in accordance with the melting point of an intermediate product; and producing terephthaloyl chloride by subjecting the hexachloro-p-xylene and terephthalic acid to a reaction in the presence of a Lewis acid catalyst.

According to an example embodiment of the present disclosure, in the method for producing hexachloro-p-xylene, a plurality of reactors may be connected in parallel.

According to an example embodiment of the present disclosure, the plurality of reactors may include a first reactor and a second reactor connected in parallel, and a process may be repeatedly performed to produce hexachloro-p-xylene without discharging unreacted chlorine gas to the outside, the process including: continuously introducing chlorine gas into the first reactor charged with p-xylene to initiate a reaction while discharging hydrogen chloride gas that is a by-product generated according to conversion of hexachloro-p-xylene to the outside; transferring, at a conversion rate of hexachloro-p-xylene at which chlorine gas that is continuously introduced into the first reactor in which the reaction is initiated begins to be discharged as an unreacted material, unreacted chlorine gas that is continuously introduced into the first reactor and does not participate in the reaction to the second reactor without being discharged to the outside, so as to allow the transferred unreacted chlorine gas to first react with p-xylene charged into the second reactor; after the reaction in the first reactor is terminated, connecting the chlorine gas that is continuously introduced to the first reactor to the second reactor so as to start continuous introduction of the chlorine gas into the second reactor; and while the first reactor from which the product is discharged is charged with p-xylene, at a conversion rate of hexachloro-p-xylene at which chlorine gas that is continuously introduced into the second reactor begins to be discharged as an unreacted material, transferring the unreacted chlorine gas to the first reactor so as to allow the transferred unreacted chlorine gas to first react with the p-xylene charged into the first reactor.

According to an example embodiment of the present disclosure, the method for producing terephthaloyl chloride may further include purifying the produced hexachloro-p-xylene.

According to an example embodiment of the present disclosure, the purifying may be performed using a distillation column.

According to an example embodiment of the present disclosure, hexachloro-p-xylene may be purified in the distillation column at a bottom temperature of 100 to 300°C, a top temperature of 100 to 250°C, and a pressure of 1 to 20 torr.

According to an example embodiment of the present disclosure, the Lewis acid catalyst may be one or two or more selected from aluminum trichloride, zinc chloride, and ferric chloride.

According to an example embodiment of the present disclosure, the method for producing terephthaloyl chloride may further include purifying the produced terephthaloyl chloride.

According to an example embodiment of the present disclosure, the purifying may be performed using a distillation column.

According to an example embodiment of the present disclosure, hexachloro-p-xylene may be purified in the distillation column at a controlled distillation temperature of 75 to 250°C and a pressure of 1 torr to 20 torr.

### [Advantageous Effects]

According to the present disclosure, hexachloro-p-xylene having a low impurity content may be obtained by performing photo-irradiation instead of using a radical initiator.

In addition, according to the present disclosure, by dividing the reaction temperature into a plurality of ranges and gradually increasing the reaction temperature in each range during the photo-reaction, a relatively high solubility of chlorine gas may be maintained within an appropriate viscosity range, thereby producing hexachloro-p-xylene with a high yield.

According to the present disclosure, by introducing a chlorine gas circulation system that circulates unreacted chlorine gas to the first reactor and the second reactor during the production of hexachloro-p-xylene as an intermediate material, hexachloro-p-xylene may be produced without discharging toxic chlorine gas to the outside.

### [Description of Drawings]

FIG. 1 is a schematic diagram illustrating the steps for producing terephthaloyl chloride.
FIG. 2 is a schematic diagram illustrating the steps for producing hexachloro-p-xylene.

### [Best Mode]

The present disclosure will be described in more detail through specific examples or embodiments including the following accompanying drawings. However, the following specific examples or embodiments are only references for describing the present disclosure in detail, and the present disclosure is not limited thereto and may be implemented in various forms.

In addition, unless otherwise defined, all the technical terms and scientific terms used have the same meanings as commonly understood by those skilled in the art to which the present disclosure pertains. The terms used in the present disclosure are merely used to effectively describe a specific embodiment, but are not intended to limit the present disclosure.

In addition, unless the context clearly indicates otherwise, singular forms used in the specification and the appended claims may be intended to include plural forms.

In addition, unless explicitly described to the contrary, "comprising" any components will be understood to imply further inclusion of other components rather than the exclusion of any other components.

In addition, in the present disclosure, a chlorinated intermediate product described below may be any one of monochloro-p-xylene, dichloro-p-xylene, trichloro-p-xylene, tetrachloro-p-xylene, or pentachloro-p-xylene.

Hexachloro-p-xylene, which is a final product formed by the chlorination reaction of the present disclosure, is produced by a substitution reaction in which chlorine radicals generated by irradiating chlorine gas with ultraviolet light react with hydrogens of methyl groups of p-xylene.

However, as the concentration of hexachloro-p-xylene increases through the photo-reaction, the viscosity also increases, resulting in a decrease in the reaction rate.

On the other hand, when the reaction is performed at a high reaction temperature, the viscosity is low, but the solubility of chlorine gas in liquid p-xylene becomes extremely low. Accordingly, the time for participating in the reaction becomes extremely short, which may cause a problem in that chlorine gas is discharged to the outside before reacting with p-xylene.

Accordingly, the present disclosure has been achieved to solve the above problems that occur when the reaction is performed while increasing the reaction temperature as described above, by performing the reaction in a stepwise manner divided into four or more stages, and preferably ten or more stages, in which, as the degree of chlorination of p-xylene increases, the melting point of the product increases due to the formation of an intermediate product generated accordingly, and when an intermediate product having an increased melting point is generated, the reaction is performed by increasing the reaction temperature to a temperature between a melting start temperature and a melting end temperature of a mixture containing the generated intermediate product.

The present disclosure provides a new reaction method that adopts an environmentally friendly means in which, as the conversion rate of the intermediate product increases, when the product is collected and its melting point is measured, the process between the melting start point, at which melting begins, and the melting end point, at which melting is completed, is divided into as many stages as possible, and as the number of stages of the reaction increases, the solubility of chlorine gas is increased to induce chlorine gas to participate in the reaction as much as possible, thereby substantially preventing or minimizing the release of the entire chlorine gas to the outside.

Specifically, during the reaction, chlorination proceeds at the methyl groups of p-xylene, and chlorinated products are generated. The chlorinated products have higher melting points than p-xylene, and as the chlorination of p-xylene further proceeds, the melting points of the chlorinated products also increase. For example, the melting point of trichloro-p-xylene is higher than that of monochloro-p-xylene, and the melting point of pentachloro-p-xylene is higher than that of trichloro-p-xylene.

Accordingly, the present disclosure provides a new environmentally friendly method for producing hexachloro-p-xylene, which recognizes that the melting point increases due to a mixture of various types of chlorinated intermediate products or final products generated depending on the conversion rate of p-xylene resulting from chlorination of p-xylene, and performs the reaction while continuously increasing the reaction temperature in accordance with the increase in the melting point, thereby enabling chlorine to be used in the reaction without being discharged to the outside.

In the present disclosure, the reaction is performed while the temperature is increased by dividing the temperature range from a low temperature to a high temperature into at least three stages, four stages, five stages, six stages, seven stages, eight stages, nine stages, or ten or more stages, thereby controlling the solubility of chlorine gas while maintaining the viscosity at a constant level, enabling a rapid reaction rate and allowing chlorine to participate in the reaction to the maximum extent without being released to the outside, and thus achieving a high yield of 95% or higher.

In one specific example embodiment according to the present disclosure, the reaction is initiated under conditions in which the reaction temperature is increased from 13°C to 20°C for 4 hours. As the conversion proceeds, the reaction is performed at 47°C to 60°C for 4 hours, at 63°C to 67°C for 4 hours, at 67°C to 83°C for 14 hours, at 87°C to 99°C for 2 hours, and at 106°C to 113°C for 2 hours, thereby converting 95% or more, and preferably 98% or more, of the material into the product without release of chlorine gas to the outside.

In addition, the present disclosure adopts a means for preventing or minimizing the release of chlorine gas to the outside by combining a step of circulating unreacted chlorine gas from the first reactor to the second reactor or from the second reactor to the first reactor, together with multi-stage reaction temperatures corresponding to the melting point of the product.

By means of the present disclosure, the inventors have solved the problem that occurs when, as in the conventional case, the photo-reaction is directly performed after increasing the temperature to a level at which all chlorinated intermediate products or final products may be melted.

That is, according to the present disclosure, it has been found that, due to the high temperature, the reaction becomes difficult to control, particularly, the solubility of chlorine gas used in the reaction decreases, resulting in a reduction in reaction efficiency. In addition, the problem of having to discharge unreacted chlorine gas to the outside may be eliminated or minimized.

The present disclosure has achieved the above object by employing a multi-stage reaction temperature means for performing the reaction while gradually increasing the temperature from the melting point of p-xylene to the melting points of the chlorinated products, and/or by employing first and second reactors connected in parallel, in which chlorine gas introduced into one reactor reacts therein, and unreacted chlorine gas remaining after the reaction is circulated to the other reactor to participate in the reaction.

In the present reaction, by adopting the above temperature range and the circulating reaction means of the reactive chlorine gas, there is an advantage in that the chlorination reactivity of p-xylene may be maintained at a high level even when a mixed gas of chlorine and hydrogen chloride, which is a by-product generated during the reaction, is used.

The circulation of the chlorine gas will be described in detail.

In the photo-reaction for producing hexachloro-p-xylene using p-xylene, as the reaction proceeds, the reaction rate of chlorine gas decreases due to the gradual increase in steric effects of the methyl groups substituted by chlorine, thereby reducing the rate of additional chlorination reactions and causing an increase in the content of unreacted chlorine gas.

Accordingly, the present disclosure adopts a means in which a plurality of reactors are connected in parallel, and unreacted chlorine gas sequentially discharged from the plurality of reactors is transferred and circulated to another reactor, such that the unreacted chlorine gas is not discharged to the outside but is instead transferred to another reactor to participate in the reaction.

The plurality of reactors may be two or three or more, but is not limited thereto.

Hereinafter, the present disclosure will be described in more detail using reactors in which two reactors are connected in parallel.

In the present disclosure, first, chlorine gas is continuously introduced into a first reactor charged with p-xylene to initiate a reaction, while hydrogen chloride gas, which is a by-product generated depending on the conversion of hexachloro-p-xylene, is discharged to the outside. In this case, the introduced chlorine gas reacts quantitatively with p-xylene and is consumed in the reaction. Subsequently, at a conversion rate of hexachloro-p-xylene at which chlorine gas that is continuously introduced into the first reactor begins to be discharged as an unreacted material, unreacted chlorine gas that is continuously introduced into the first reactor and does not participate in the reaction is, at the time when it is discharged from the first reactor, transferred to the second reactor without being discharged to the outside, so that the transferred unreacted chlorine gas initiates a first reaction with p-xylene charged into the second reactor. When the reaction in the first reactor is completed, the introduction of the circulating chlorine gas into the first reactor is stopped, and the chlorine gas introduced into the first reactor is connected to the second reactor so that the chlorine gas is continuously introduced into the second reactor to participate in the reaction.

Subsequently, the process of circulating unreacted chlorine gas to the first reactor so that the circulated chlorine gas initiates a first reaction with the p-xylene charged into the first reactor at a conversion rate of hexachloro-p-xylene at which chlorine gas that is continuously introduced into the second reactor begins to be discharged as an unreacted material, while the first reactor from which the product is discharged is charged again with p-xylene, which is the starting material, is repeated, thereby producing hexachloro-p-xylene without discharging unreacted chlorine gas to the outside.

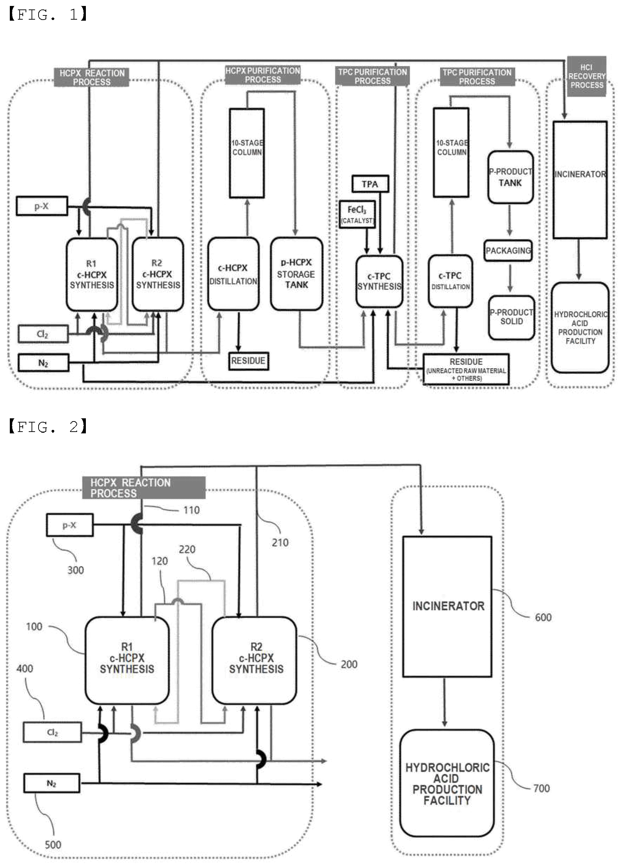

Hereinafter, the method for producing hexachloro-p-xylene will be described in more detail with reference to FIG. 2, divided into steps.

According to an example embodiment of the present disclosure, the method for producing hexachloro-p-xylene may include:
S1) introducing and charging p-xylene into a first reactor 100 and a second reactor 200;
S2) continuously introducing chlorine gas into the first reactor 100, the introduced chlorine gas reacting 100% with the p-xylene, and transferring hydrogen chloride gas, which is a by-product generated from the reaction, to an incinerator 600 through a first transfer line 110 to recover hydrochloric acid;
S3) when the reaction of chlorine gas in the first reactor 100 decreases to less than 100% and unreacted chlorine gas is generated, closing the first discharge transfer line 110;
S4) after closing the first discharge transfer line 110, transferring unreacted chlorine gas and residual hydrogen chloride gas in the first reactor 100 to the second reactor 200 through a first cross-transfer line 120 so that the transferred gases are first reacted in the second reactor 200 to produce hexachloro-p-xylene simultaneously in the first reactor 100 and the second reactor 200;
S5) transferring hydrogen chloride gas generated as a by-product in the second reactor 200 to an incinerator 600 through a second discharge transfer line 210 provided at an upper portion of the second reactor 200;
S6) when the reaction in the first reactor 100 is completed, closing the first cross-transfer line 120 and switching the introduction of chlorine gas that has been introduced into the first reactor 100 to the second reactor 200;
S7) discharging hexachloro-p-xylene, which is a product, from the first reactor 100 and introducing fresh p-xylene;
S8) when the reaction of chlorine gas in the second reactor 200 decreases to less than 100% and unreacted chlorine gas is generated, closing the second discharge transfer line 210 and introducing unreacted chlorine gas and residual hydrogen chloride remaining in the second reactor 200 into the first reactor 100 through a second cross-transfer line 220 so that the reaction for producing hexachloro-p-xylene is simultaneously performed in the second reactor 200 and the first reactor 100;
S9) after the reaction in the second reactor 200 is completed, introducing chlorine gas that is introduced into the second reactor 200 into the first reactor 100 to allow the reaction to proceed, and opening the first discharge transfer line 110 to transfer hydrogen chloride, which is a by-product, to the incinerator 600 to recover hydrochloric acid; and
S10) discharging hexachloro-p-xylene from the second reactor 200 and introducing fresh p-xylene into the second reactor 200, and steps S2 to S10 may be repeated to continuously produce hexachloro-p-xylene.

In the step of producing hexachloro-p-xylene, chlorine gas and p-xylene are converted into hexachloro-p-xylene through a photo-reaction. The chlorine gas is decomposed into chlorine radicals by the photo-reaction, the chlorine radicals are substituted for hydrogens of methyl groups of p-xylene to produce hexachloro-p-xylene, and hydrogen chloride is discharged as a by-product.

Through the method for producing hexachloro-p-xylene, chlorine gas may be completely consumed in the reaction, thereby preventing chlorine gas, which is fatal to the human body or the environment, from being released to the outside.

In addition, in the method for producing hexachloro-p-xylene, when the reaction is performed within the plurality of temperature ranges, there is an advantage in that high reactivity may be maintained even when the mixed gas of chlorine and hydrogen chloride is used.

In addition, according to an example embodiment of the present disclosure, the photo-reaction may include an ultraviolet light intensity range of less than 2,000 LUX, specifically 100 to 1,800 LUX, and more specifically 100 to 1,500 LUX.

Conventionally, the light intensity used for the photo-reaction was in a high-energy range of 2,000 LUX to 50,000 LUX; however, in such light intensity ranges, side reactions could occur, resulting in a disadvantage of a low yield of hexachloro-p-xylene.

Accordingly, the inventors found that hexachloro-p-xylene could be produced even when the intensity of the radiated ultraviolet light was controlled to be in the range of 100 to less than 1,500 LUX, and that, when hexachloro-p-xylene was produced within the above light intensity range, side reactions were reduced and the yield was high.

In addition, when the reaction is performed within the above light intensity range, the reaction heat generated according to the reaction is low, making it extremely easy to control the temperature while also allowing the reaction rate to be controlled.

In addition, although the irradiation area of the photo-reaction is not limited as long as the reaction proceeds smoothly, the irradiation area of the photo-reaction may be specifically 5 to 30 cm², more specifically 10 to 20 cm², and still more specifically, 10 to 15 cm² per kilogram of p-xylene.

Previously, a mercury lamp was used as a light source for the photo-reaction; however, in the case of the mercury lamp, the short-wavelength light of a low-pressure mercury lamp may cause other photochemical side reactions, thereby lowering the purity of the product, whereas the long-wavelength light of a high-pressure mercury lamp is not sufficient to induce a chlorine radical reaction, resulting in higher energy consumption. In addition, when the mercury lamp is used, more heat may be generated, and a problem may occur in that an additional cooling device needs to be introduced to reduce the heat.

Accordingly, the present disclosure may overcome the disadvantages of the mercury lamp by using an LED lamp. By using the LED lamp, the wavelength range suitable for the reaction may be controlled, thereby reducing photochemical side reactions, and heat is not generated compared with the mercury lamp even during long-term use, such that an additional cooling device is not required. In addition, the LED lamp has low power consumption, which provides an advantage of high energy efficiency.

In particular, in the case of a mixed gas in which chlorine gas and hydrogen chloride gas introduced into the reactor through the first cross-transfer line or the second cross-transfer line are mixed, the probability of occurrence of side reactions may be higher at high light intensity than that of a single chlorine gas, and thus the reactivity may decrease.

Accordingly, in the present disclosure, by performing irradiation with light of less than 2,000 LUX, hexachloro-p-xylene with a high yield may be obtained while reducing the above side reactions.

In addition, by simultaneously satisfying the above light intensity range and the plurality of temperature ranges, hexachloro-p-xylene with an extremely high yield may be obtained while side reactions are significantly reduced and the discharge of hydrogen chloride gas, which is a by-product, is smoothly performed.

The discharged hydrogen chloride gas is transferred to the incinerator, where all unreacted chlorine gas is discharged, and the hydrogen chloride gas enters a hydrochloric acid production facility to produce a 35% hydrochloric acid solution.

Specifically, the hydrogen chloride gas generated in the incinerator is dissolved in water through a scrubbing system in the hydrochloric acid production facility to produce hydrochloric acid.

The produced 35% hydrochloric acid solution is transferred to a hydrochloric acid tank for storage.

According to an example embodiment of the present disclosure, the produced hexachloro-p-xylene and terephthalic acid may be reacted to produce terephthaloyl chloride.

As an example embodiment, the step of producing terephthaloyl chloride may include: producing hexachloro-p-xylene from p-xylene; purifying the produced hexachloro-p-xylene; and reacting the purified p-xylene and terephthalic acid in the presence of an acid catalyst to produce terephthaloyl chloride.

In addition, as an example embodiment, the step of producing terephthaloyl chloride includes: producing hexachloro-p-xylene from p-xylene; purifying the produced hexachloro-p-xylene; reacting the purified p-xylene and terephthalic acid in the presence of a Lewis acid catalyst to produce terephthaloyl chloride; and purifying the produced terephthaloyl chloride.

First, the step of purifying hexachloro-p-xylene may include a recrystallization method, a rectification method, a distillation method, and the like, and the distillation method may include a vacuum distillation method or a molecular distillation method.

In the purification step, the distillation method may be performed using a distillation column, and the distillation column is not limited as long as it is a device generally used for distilling compounds.

The distillation column may purify hexachloro-p-xylene under conditions in which a bottom temperature of the distillation is 100 to 300°C, a top temperature is 100 to 250°C, and a pressure is 1 to 20 torr, but is not limited thereto.

In addition, the catalyst used in the step of producing terephthaloyl chloride by reacting the purified hexachloro-p-xylene with terephthalic acid in the presence of an acid catalyst may be a Lewis acid catalyst.

The Lewis acid catalyst may include one or two or more selected from aluminum trichloride, zinc chloride, and ferric chloride, but is not limited thereto as long as it is a catalyst commonly used in the synthesis.

In the synthesis of terephthaloyl chloride, terephthalic acid may be introduced in an amount of 30 to 300 parts by weight, preferably 40 to 200 parts by weight, and more preferably 45 to 130 parts by weight with respect to 100 parts by weight of hexachloro-p-xylene, but is not limited thereto.

In addition, the acid catalyst may be included in an amount of 0.001 to 10 parts by weight, preferably 0.003 to 1 part by weight, and more preferably 0.005 to 0.01 parts by weight with respect to 100 parts by weight of hexachloro-p-xylene, but is not limited thereto.

In addition, according to an example embodiment of the present disclosure, the method for producing terephthaloyl chloride may further include a purification step, and the purification step of terephthaloyl chloride may include a recrystallization method, a rectification method, a distillation method, and the like, and the distillation method may include a vacuum distillation method or a molecular distillation method.

In the purification step of terephthaloyl chloride, the distillation method may be performed using a distillation column, and the distillation column is not limited as long as it is a device generally used for distilling compounds.

The distillation column may purify terephthaloyl chloride at a controlled distillation temperature of 75 to 250°C and a pressure of 1 torr to 20 torr, but is not limited thereto.

Hereinafter, the present disclosure will be described in more detail based on the following Examples and Comparative Examples. However, the following Examples and Comparative Examples are only examples for explaining the present disclosure in more detail, and the present disclosure is not limited by the following Examples and Comparative Examples.

### [Example 1]

### 1) Step of producing hexachloro-p-xylene

After 100 parts by weight of p-xylene was introduced from a p-xylene supply unit 300 into each of a first reactor 100 and a second reactor 200, nitrogen was injected from a nitrogen gas supply unit 500 into the first reactor 100 to remove air inside, and then, chlorine gas was injected from a chlorine gas supply unit 400 into the first reactor 100 and subjected to a photo-reaction, thereby producing hexachloro-p-xylene.

The chlorine gas was injected at a rate of 30 parts by weight per hour with respect to 100 parts by weight of the p-xylene.

In the photo-reaction, as the reaction proceeded in the first reactor 100, intermediate products were generated depending on the conversion rates shown in Table 1. As the intermediate products were formed, the melting point of the intermediate product mixture increased, and the reaction was performed under multi-stage temperature-increasing conditions at the temperatures and times shown in Table 2. During the reaction, stirring was performed at 300 rpm, and the photo-reaction was performed by performing irradiation with ultraviolet light of 1,000 LUX onto a light irradiation area of 15 cm² per 1 kg of raw material.

In addition, the external chlorine gas introduced into the first reactor was quantitatively consumed by participating in the reaction at the initial stage of the reaction, and hydrogen chloride, which is a by-product, was discharged to an incinerator 600 through a first discharge transfer line 110. When the yield of hexachloro-p-xylene reached 0.1%, unreacted chlorine gas began to be discharged to the outside, and at this time, the unreacted chlorine gas was circulated to the second reactor to initiate a chlorination reaction in the second reactor under the same conditions as those in the first reactor.

The reaction was performed in the first reactor under the conditions shown in Tables 1 and 2, and was terminated after 32 hours. A chlorine gas introduction line of the first reactor was then connected to a chlorine gas introduction line of the second reactor, and chlorine gas was introduced into the second reactor. The reaction of the second reactor was performed under the conditions of conversion rate, reaction temperature, and reaction time as shown in Tables 3 and 4.

When unreacted chlorine gas was generated at the point where the yield of hexachloro-p-xylene reached 0.1% in the second reactor, the chlorine gas was transferred to the first reactor to initiate a chlorination reaction in the first reactor.

**[Table 1]**

| **Elapsed reaction time (H)** | **MP (°C)** | |
|---|---|---|
| | **Start point (Melting start point)** | **End point (Melting end point)** |
| **0** | **13** | |
| **2** | **13 ~ 20** | |
| **4** | | |
| **8** | **47** | **60** |
| **12** | **56** | **64** |
| **16** | **63** | **67** |
| **20** | **65** | **70** |
| **24** | **66** | **71** |
| **28** | **67** | **83** |
| **30** | **87** | **99** |
| **32** | **106** | **113** |

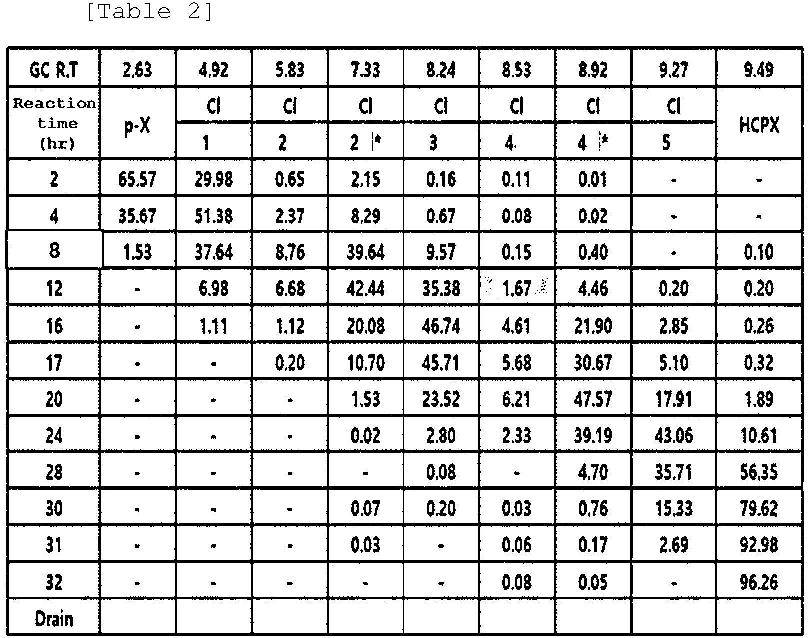

### 2) Step of purifying hexachloro-p-xylene

The hexachloro-p-xylene produced in the first reactor 100 and the second reactor 200 was transferred to the bottom of a first distillation column. In order to purify the hexachloro-p-xylene, the first distillation column was operated at a total column pressure of 5 torr, a bottom temperature of 160°C, a top temperature of 150°C, and a stirring speed of 300 rpm, the hexachloro-p-xylene was purified at high purity and separated from the hexachloro-p-xylene residue, and then, the purified hexachloro-p-xylene was transferred to a first storage tank for storage.

### 3) Step of reacting to produce terephthaloyl chloride

In the step of purifying hexachloro-p-xylene, the high-purity hexachloro-p-xylene stored in the first storage tank was transferred to a third reactor, and terephthalic acid and an iron(III) chloride (FeCl₃) catalyst were introduced into the third reactor and reacted for 5 hours to produce terephthaloyl chloride.

In the reaction, 100 parts by weight of terephthalic acid and 0.005 parts by weight of FeCl₃ were mixed and reacted with respect to 100 parts by weight of high-purity hexachloro-p-xylene, and the reaction was performed at a temperature of 120°C under atmospheric pressure.

### 4) Step of purifying terephthaloyl chloride

The terephthaloyl chloride produced in 3) Step of reacting to produce terephthaloyl chloride was transferred to a second distillation column and was primarily purified. The purification of the second distillation column was performed under the conditions in which a total column pressure was 5 torr, a bottom temperature was 160°C, a top temperature was 130°C, and a stirring speed was 300 rpm. The highly purified terephthaloyl chloride was transferred to a second storage tank and stored.

Hereinabove, although the present disclosure has been described by specific matters, limited embodiments, and the drawings, they have been provided only for assisting in the entire understanding of the present disclosure. Therefore, the present disclosure is not limited to the embodiments, and various modifications and changes may be made by those skilled in the art to which the present disclosure pertains from this description.

Therefore, the spirit of the present disclosure should not be limited to the described embodiments, but the claims and all modifications equal or equivalent to the claims are intended to fall within the spirit of the present disclosure.

### [Detailed Description of Main Elements]

100: First reactor
110: First discharge transfer line
120: First cross-transfer line
200: Second reactor
210: Second discharge transfer line
220: Second cross-transfer line
300: p-Xylene supply unit
400: Chlorine gas supply unit
500: Inert gas supply unit
600: Incinerator
700: Hydrochloric acid production facility

## Claims

1. A method for producing hexachloro-p-xylene by subjecting p-xylene and chlorine gas to a photo-reaction, wherein the photo-reaction is performed in a plurality of four or more temperature ranges in accordance with an increase in the melting point of an intermediate product generated depending on the conversion rate of hexachloro-p-xylene.

2. The method of claim 1, wherein a plurality of reactors are connected in parallel.

3. The method of claim 2, wherein the plurality of reactors include a first reactor and a second reactor connected in parallel, and a process is repeatedly performed to produce hexachloro-p-xylene without discharging unreacted chlorine gas to the outside, the process including: continuously introducing chlorine gas into the first reactor charged with p-xylene to initiate a reaction while discharging hydrogen chloride gas that is a by-product generated according to conversion of hexachloro-p-xylene to the outside; transferring, at a conversion rate of hexachloro-p-xylene at which chlorine gas that is continuously introduced into the first reactor in which the reaction is initiated begins to be discharged as an unreacted material, unreacted chlorine gas that is continuously introduced into the first reactor and does not participate in the reaction to the second reactor without being discharged to the outside, so as to allow the transferred unreacted chlorine gas to first react with p-xylene charged into the second reactor; after the reaction in the first reactor is terminated, connecting the chlorine gas that is continuously introduced to the first reactor to the second reactor so as to start continuous introduction of the chlorine gas into the second reactor; and while the first reactor from which the product is discharged is charged with p-xylene, at a conversion rate of hexachloro-p-xylene at which chlorine gas that is continuously introduced into the second reactor begins to be discharged as an unreacted material, transferring the unreacted chlorine gas to the first reactor so as to allow the transferred unreacted chlorine gas to first react with the p-xylene charged into the first reactor.

4. A method for producing terephthaloyl chloride by reacting hexachloro-p-xylene with terephthalic acid, the method comprising:
producing hexachloro-p-xylene by subjecting p-xylene and chlorine gas to a photo-reaction in four or more temperature ranges in accordance with the melting point of an intermediate product; and
producing terephthaloyl chloride by subjecting the hexachloro-p-xylene and terephthalic acid to a reaction in the presence of a Lewis acid catalyst.

5. The method of claim 4, wherein in the step of producing hexachloro-p-xylene, a plurality of reactors are connected in parallel.

6. The method of claim 5, wherein the plurality of reactors include a first reactor and a second reactor connected in parallel, and a process is repeatedly performed to produce hexachloro-p-xylene without discharging unreacted chlorine gas to the outside, the process including: continuously introducing chlorine gas into the first reactor charged with p-xylene to initiate a reaction while discharging hydrogen chloride gas that is a by-product generated according to conversion of hexachloro-p-xylene to the outside; transferring, at a conversion rate of hexachloro-p-xylene at which chlorine gas that is continuously introduced into the first reactor in which the reaction is initiated begins to be discharged as an unreacted material, unreacted chlorine gas that is continuously introduced into the first reactor and does not participate in the reaction to the second reactor without being discharged to the outside, so as to allow the transferred unreacted chlorine gas to first react with p-xylene charged into the second reactor; after the reaction in the first reactor is terminated, connecting the chlorine gas that is continuously introduced to the first reactor to the second reactor so as to start continuous introduction of the chlorine gas into the second reactor; and while the first reactor from which the product is discharged is charged with p-xylene, at a conversion rate of hexachloro-p-xylene at which chlorine gas that is continuously introduced into the second reactor begins to be discharged as an unreacted material, transferring the unreacted chlorine gas to the first reactor so as to allow the transferred unreacted chlorine gas to first react with the p-xylene charged into the first reactor.

7. The method of claim 4, further comprising purifying the produced hexachloro-p-xylene.

8. The method of claim 7, wherein the purifying is performed using a distillation column.

9. The method of claim 8, wherein hexachloro-p-xylene is purified in the distillation column at a bottom temperature of 100 to 300°C, a top temperature of 100 to 250°C, and a pressure of 1 to 20 torr.

10. The method of claim 4, wherein the Lewis acid catalyst is one or two or more selected from aluminum trichloride, zinc chloride, and ferric chloride.

11. The method of claim 4, further comprising purifying the produced terephthaloyl chloride.

12. The method of claim 11, wherein the purifying is performed using a distillation column.

13. The method of claim 12, wherein hexachloro-p-xylene is purified in the distillation column at a controlled distillation temperature of 75 to 250°C and a pressure of 1 torr to 20 torr.
